# EUROPEAN PATENT APPLICATION

(11) **EP 2 194 034 A1**
(43) Date of publication of application: **09.06.2010**
(21) Application number: 08170449.6
(22) Date of filing: 02.12.2008
(51) Int. Cl.: C07C 6/04, C07C 6/06

(54) **Disproportionation process**

(71) Applicant: Shell Internationale Research Maatschappij B.V., 2596 HR Den Haag (NL)
(72) Inventor: De Boer, Eric Johannes Maria, 1031HW Amsterdam (NL); Van Der Heijden, Harry, 1031HW Amsterdam (NL); Van Der Steen, Frederik Hendrik, 1031HW Amsterdam (NL); Van Zon, Arie, 1031HW Amsterdam (NL)
(74) Representative: Matthezing, Robert Maarten

(57) **Abstract**

According to the present invention, there is provided a process for the disproportionation of olefinic hydrocarbons, said process comprising contacting at least one olefinic hydrocarbon with a catalyst comprising at least one of molybdenum and rhenium supported on an inorganic oxide support in the presence of a promoter selected from organoborane and organoaluminium compounds, wherein said process is carried out at a temperature in the range of from 100 to 200 °C.

## Description

### Field of the Invention

The present invention relates to a process for the disproportionation of olefinic hydrocarbons.

### Background of the Invention

Reactions of olefinic molecules in the presence of metal-containing catalysts to produce other olefinic molecules are known in the art as 'disproportionation' reactions.

The olefin disproportionation reaction can be visualized as the breaking of two existing double bonds and the formation of two new double bonds. A typical olefin disproportionation process is illustrated in US 3,261,879, wherein two similar non-symmetrical olefin molecules react in the presence of certain catalysts to produce one olefin of a higher carbon number and one olefin of a lower carbon number. For example, propylene disproportionates by the process of US 3,261,879 to produce ethylene and butylenes.

A variation of this disproportionation process is termed 'reverse disproportionation' and is illustrated by the NL 6514985, wherein, in one modification, molecules of two dissimilar olefins are reacted to form two molecules of a single olefin product, e.g., ethylene and 2-butene react to form propylene.

'Ring opening disproportionation' is disclosed in NL 6702703, wherein a cyclic olefin and an acyclic olefin react to form a single product molecule. For example, ethylene reacts with cyclopentene by ring opening disproportionation to produce 1,6-heptadiene.

As used herein, the terms `disproportionation' and 'disproportionation process' are meant to include all variations of disproportionations. Such processes are also known in the art as 'metathesis'.

A variety of catalysts have been employed for conducting disproportionation reactions, such as those disclosed in US 3,340,322, US 3,637,892, US 3,760,026, US 3,792,108, US 3,872,180, and GB 1,128,091. Among the catalysts that have been developed for disproportionation include inorganic refractory materials containing molybdenum and/or tungsten oxide.

Several patents disclose the use of promoter(s) to enhance disproportionation catalyst activity. Elemental metal promoters selected from the group consisting of barium, magnesium, tungsten, silicon, antimony, zinc, manganese and tin are disclosed in US 4,568,788, US 4,522,936, US 4,5914,235, and US 4,629,719. In addition, organometallic compounds, such as aluminium and tin alkyls to promote solid catalysts including molybdenum and rhenium oxide for the disproportionation are disclosed in US 4,454,368 and US 3,829,523.

US 5,254,786, US 5,098,876 and US 5,057,644 disclose the use of organoborane compounds as promoters for disproportionation catalysts comprising rhenium and/or molybdenum supported on an inorganic oxide support. Such organoborane-promoted catalysts have been shown to increase the activity and selectivity of disproportionation reactions carried out at ambient temperature.

Regardless of the advances described above, there still remains problems associated with the industrial scale disproportionation of olefins. Not least of these problems is the concurrent dimerisation of olefins which leads to the production of non-reactive tri- and tetra-substituted internal olefins. The formation and presence of these non-reactive olefins causes problems due to their presence as non-reactive diluents and leads to slower rates of disproportionation and increased bleed streams. Further, it has been found that non-reactive high molecular weight branched olefins inactivate the inorganic supported rhenium and/or molybdenum catalysts by blocking catalyst pores and covering the catalyst surface. Such inactivation leads to shorter catalyst lifetimes.

It is an object of the present invention to provide an olefin disproportionation process with high activity and efficiency, in which the dimerisation of olefins and the formation of non-reactive tri- and tetra-substituted internal olefins are reduced.

### Summary of the Invention

According to the present invention, there is provided a process for the disproportionation of olefinic hydrocarbons, said process comprising contacting at least one olefinic hydrocarbon with a catalyst comprising at least one of molybdenum and rhenium supported on an inorganic oxide support in the presence of a promoter selected from organoborane and organoaluminium compounds, wherein said process is carried out at a temperature in the range of from 100 to 200 °C.

### Detailed Description of the Invention

It has now surprisingly been found that a highly active, efficient and selective process for the disproportionation of olefins can be achieved with reduced levels of dimerisation and formation of non-reactive tri- and tetra-substituted internal olefins by contacting one or more olefinic hydrocarbons with a disproportionation catalyst comprising at least one of molybdenum and rhenium supported on an inorganic oxide support, in the presence of a promoter selected from organoborane and organoaluminium compounds, at a temperature in the range of from 100 to 200 °C.

Olefinic hydrocarbons which are suitable for use as a feed stream for the disproportionation process of the present invention are non-tertiary, non-conjugated acyclic monoolefins and polyolefins having at least 2 carbon atoms per molecule including cycloalkyl, cycloalkenyl and aryl derivatives thereof; cyclic and polycyclic monoolefins and polyolefins having at least 3 carbon atoms per molecule including alkyl and aryl derivatives thereof; mixtures of the above olefins; and mixtures of ethylene and the above olefins. Preferably, the olefinic hydrocarbons are acyclic monoolefins.

A useful group of olefinic hydrocarbons are acyclic olefins leaving carbon numbers ranging from C₂ to about C₅₀, preferably from C₃ to about C₃₀, and cyclic olefins having carbon numbers ranging from C₄ to about C₃₀. Non-tertiary olefins are those olefins wherein each carbon atom, which is attached to another carbon number by means of a double bond, is also attached to at least one hydrogen atom.

Some specific examples of acyclic olefins suitable for disproportionation according to this invention are acyclic 1- and 2-alkenes, other internal alkenes, and alkyl and aryl derivatives thereof having from 2 to 30 carbon atoms per molecule. Some specific examples of such olefins are propylene, 1-butene, 2-butene, 1-pentene, 2-pentene, 1-hexene, 2-hexene, 3-hexene, 2-heptene, 1-octene, 4-octene, 2-nonene, 5-decene, 1-dodecene, 2-tetradecene, 1-hexadecene, 2-methyl-1-butene, 3-methyl-1-butene, 1-phenylbutene-2, 3-heptene and the like, and mixtures thereof.

Some specific examples of cyclic olefins suitable for disproportionation according to this invention are cyclobutene, cyclopentene, cycloheptene, cyclooctene, 5-n-propylcyclooctene, cyclodecene, cyclododecene, 3,3,5,5-tetramethylcyclononene, 3,4,5,6,7-pentaethylcyclodecene, 1,5-cyclooctadiene, 1,5,9-cyclododecatriene, 1,4,7,10-cyclododecatetraene, 6-methyl-6-ethylcyclooctadiene-1,4 and the like, and mixtures thereof.

The olefinic hydrocarbon-containing feed stream should be essentially free of impurities which adversely affect the reaction. Such impurities generally poison, or deactivate, the catalyst. A subsequent reactivation of the catalyst to remove the effect of such impurities can be made by heat treatment with air, using an inert gas to control burn-off temperature.

The catalyst used in the present invention is prepared by forming a calcined composite comprising at least one of molybdenum and rhenium supported on an inorganic oxide support and contacting the calcined composite with a promoting amount of an organoborane compound. The inorganic oxide support comprises a solid usually containing a major proportion of silica or alumina. Such materials are commonly known as refractory oxides and include synthetic products as well as acid-treated clays or the crystalline alumina silicates known in the art as molecular sieves. Synthetic refractory oxides include silica, alumina, silica-alumina, silica-magnesia, silica-titania, alumina-titania, alumina-magnesia, boria-alumina-silica, alumina-zirconia, thoria and silica-titania-zirconia. Preferred inorganic oxide supports are alumina refractory oxides, i.e., refractory oxides containing a substantial proportion of alumina, e.g., at least about 90 percent by weight of alumina. Any conventional catalytic grade of alumina including the beta or gamma forms can be used. Generally, the inorganic oxide support has a surface area of at least 10 m²/g and preferably, the surface area is from about 25 m²/g to 800 m²/g.

The molybdenum and/or rhenium can be combined with the inorganic oxide support in any conventional method such as dry mixing, ion-exchange, co-precipitation, impregnation and the like. For example, a 10-100 mesh alumina can be impregnated with an aqueous solution containing molybdenum salts, such as ammonium heptamolybdate or ammonium dimolybdate.

In a preferred embodiment, the catalyst used in the process of the present invention is a molybdenum oxide prepared by impregnating alumina with an aqueous molybdenum impregnation solution. The aqueous molybdenum solution consists of a water-soluble source of molybdenum oxide such as ammonium heptamolybdate or ammonium dimolybdate dissolved in water. Hydrogen peroxide may also be used to aid in solution preparation in some cases. For example, the molybdenum solution can be prepared by adding hydrogen peroxide to the solution in an amount in the range of from about 0.1 to about 1.0 mole of hydrogen peroxide per mole of molybdenum. Optionally, a suitable soluble amine compound such as monoethanolamine, propanol amine or ethylenediamine may be added to the molybdenum solution in order to aid in stabilization of the solution.

Following impregnation, the resulting material is dried and calcined. Drying is accomplished by conventional means. It may be carried out by forced draft drying, vacuum drying, air-drying or similar means. Drying temperatures are not critical and depend upon the particular means utilized for drying. Drying temperatures will typically range from about 50 °C. to about 150 °C.

After drying, the material is calcined to produce the finished catalyst. The material may be calcined in an oxidizing or neutral atmosphere, although air is preferred. However, if binders and/or lubricants are used the material is heated in an oxygen-containing atmosphere, preferably air, in order to burn out the binders and lubricants. Calcining temperatures will typically range from about 300 °C. to about 600 °C. Burn-out temperatures will depend on the concentration of oxygen in the burn-out atmosphere as well as the burn-out time involved. Typically, burn-out temperatures will range from about 300 °C. to about 600 °C. Drying, calcining and burn-out may be combined in one or two steps. Most frequently the calcining and/or burn-out steps are combined using an oxygen-containing atmosphere.

The final calcined composites typically contain from about 1 percent by weight to about 18 percent by weight, preferably from about 5 percent by weight to about 15 percent by weight, and more preferably from about 6 percent by weight to about 12 percent by weight molybdenum and from about 1 percent by weight to about 20 percent by weight, preferably from about 5 percent by weight to about 15 percent by weight, and more preferably from about 6 percent to about 12 percent by weight rhenium. When mixtures of molybdenum and rhenium are utilized, the final catalyst typically contains from about 1 percent by weight to about 32 percent by weight molybdenum arid/or rhenium. These types of catalysts are well known in the art and reference can be prepared according to the prior art, such as but not limited to aforementioned US 3,261,879 and US 3,365,513 for more specific details about these types of catalysts.

The supported molybdenum and/or rhenium oxide composites are preferably subjected to a pretreatment prior to contact with the promoter. While pretreatment is usually accomplished by contacting the catalyst with an oxygen-containing gas at elevated temperatures, other activation methods such as heating under a vacuum, or contact with various gases such as nitrogen or argon at high temperatures, can be used. One function served by this type of pretreatment is to convert the molybdenum and/or rhenium components into the form of the oxide if these components are not initially provided in these forms. The temperature, contact times, and other conditions of pretreatment have been reported in the prior art and are generally the same conditions which are utilized to activate a disproportionation catalyst. Typically, the pretreatment conditions include a temperature in the range of from about 300 °C. to about 900 °C. for about 30 minutes to about 24 hours. Preferably the pretreatment is carried out at a temperature of at least 600 °C.

In order to obtain the active catalyst composition used in the present invention, the molybdenum and/or rhenium oxide supported composition is treated with the promoter. The promoter can be combined with the molybdenum or rhenium oxide supported compositions in any suitable manner. For example, the molybdenum and/or rhenium oxide supported composition can be impregnated with a liquid diluent containing the promoter at ambient temperature up to 150 °C. After impregnation, the catalyst is then heated in an inert atmosphere, such as nitrogen or argon, to remove the liquid diluent. The temperature employed in removing the diluent and activating can vary widely; however, temperatures in the range of about 25 °C. to about 200 °C. are preferred. If desired, the promoter can be applied to the supported molybdenum and/or rhenium oxide in a reaction zone by spraying or otherwise contacting with the oxide. In a preferred embodiment, the promoter is introduced along with the olefins hydrocarbon containing feed stream as a means for contacting with supported molybdenum and/or rhenium oxide.

In accordance with the invention, the calcined molybdenum and/or rhenium oxide refractory materials are treated with an effective promoting amount of the promoter and heated under conditions to form a promoted catalyst. The promoter is selected from the group consisting of organoborane and organoaluminium compounds.

If the promoter is an organoborane compound, it should have at least one boron to carbon bond. Preferred organoborane compounds are alkylboranes such as triethylborane, tri-sec-butylborane, tri-n-butylborane, trimethylborane, tricyclohexylborane and the like, with tri-sec-butylborane and triethylborane being preferred.

If the promoter is an organoaluminium compound, it should have at least one aluminium to carbon bond. Preferred organoaluminium compounds are alkylaluminiums, with triethylaluminium being preferred.

Suitable molybdenum or rhenium oxide/ organoborane molar ratios are typically in the range of from about 0.1 to about 50, preferably from about 1 to about 30 and more preferably, from about 1.5 to about 25.

Suitable molybdenum or rhenium oxide/organoaluminium molar ratios are typically in the range of from about 0.1 to about 50, preferably from about 1 to about 30 and more preferably, from about 1.5 to about 25.

The process of the present invention can be carried out either batch-wise or continuously, using a fixed catalyst bed, or a stirrer equipped reactor or other mobile catalyst contacting process as well as any other well known contacting technique. Preferred reaction conditions, e.g., temperature, pressure, flow rates, etc., vary somewhat depending upon the specific catalyst composition, the particular feed olefin, desired products, etc. The process is carried out at temperatures ranging from 100 °C. to 200 °C. Preferably, the temperature is at least 110 °C. Also preferably, the temperature is no more than 180 °C, more preferably no more than 160 °C, even more preferably no more than 150 °C.

The use of such a temperature range results in a decrease in the amount of dimers formed in the disproportionation reaction, thus resulting in a more active and selective catalytic process.

The process of the present invention can suitably be carried out at pressures in the range of from 50 psig to 2000 psig. The disproportionation reaction is usually effected in a liquid phase and if desired, liquid reaction diluents are utilized. Examples of suitable diluents are hydrocarbons free from aliphatic unsaturation, such as cyclic or alicyclic alkanes of from 6 to 12 carbon atoms, i.e. hexane, isooctane and cyclohexane. Also exemplary would be monoaromatic compounds such as benzene and toluene. If the diluent is added, it is present in amounts up to 20 moles of diluent per mole of olefinic reactants.

The operable range of contact time for the process of this invention depends primarily upon the operating temperature and the activity of the catalyst, which is influenced by surface area, promoter concentration, activation temperature, etc. In general, the distribution of products is not drastically altered by variation in contact time. Shorter contact times are usually associated with higher temperatures. With proper selection of conditions and contact times, very high efficiency of conversion to desired products can be obtained.

With a fixed bed reactor, continuous flow operation at pressures in the range of 50 psig to 2000 psig, preferably in the range of from 100 psig to 1000 psig, with catalysts having densities ranging from about 0.3 grain per cc to about 2.0 gram per cc and surface areas greater than about 100 m²/g. Again the temperature of the process is in the range of from 100 to 200 °C. Preferably, the temperature is at least 110 °C. Also preferably, the temperature is no more than 180 °C, more preferably no more than 160 °C, even more preferably no more than 150 °C. Weight hourly space velocities are suitably in the range of from 0.1 to 10.0 parts by weight of olefinic hydrocarbon feed per part by weight of catalyst per hour are suitable. The space velocity is adjusted according to changes in density of feed due to change of pressure or temperature, and variation in reaction temperature and the activity of the catalyst.

In one embodiment of the present invention, desired carbon numbers of internal olefins can be prepared by disproportionation of less desirable alpha or internal olefins. By way of an example, by using the process of the present invention, 1-decene can be converted into 9-octadecene and ethylene (reaction 1) :

2 C₈H₁₇CH=C₂ --------> C₈H₁₇CH=CHC₈H₁₇ + CH₂=CH₂

and a mixture of internal decene isomers can be converted to a number of internal olefins ranging from C₃ to C₁₈. Some of these products are useful as detergent intermediates.

In another embodiment, C₃ to C₉ alpha olefins with good purity can be produced at room temperature from the disproportionation of a mixture of internal decenes and ethylene as described in reaction 2: 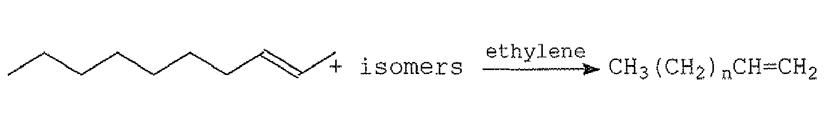 wherein n=0 to 6. Alpha olefins are used to produce a number of useful products, including comonomers for high density polyethylene (HDPE), linear low density polyethylene (LLDPE), intermediates for synthetic lube oils and lube oil additives, surfactants, paper sizings and specialty chemicals.

In another embodiment, dienes and trienes can be produced by disproportionating cyclic olefins with ethylene according to the process of the present invention. By way of illustration, cyclopentene can be reacted with ethylene to form 1,6-heptadiene, cyclooctene with ethylene to form 1,9-decadiene and 1,5-cyclooctadiene with ethylene to form 1,5-hexadiene. When this reaction is carried out according to the present invention, increased reaction rates can be achieved and undesired dimerisation reactions are minimised.

In another embodiment, cyclopentene can be reacted with propylene to produce 1,6-octadiene as the major product. While trienes and tetrenes are formed as byproducts in this disproportionation reaction, the byproducts can be recycled to produce the desired dienes, thus making the reaction quite efficient. As is true with the previously described embodiment, undesired dimerisation reactions are minimised.

The following non-limiting Examples will illustrate the invention.

### Examples

### Catalyst Activation

A glass reactor tube (2 x 20 cm) was 1/3 filled with fresh SMO-2 (a 10-15% MoO₃ on AX-200 catalyst, available from CRI Catalyst) and placed in a calcination oven. A nitrogen flow of <50 ml/minute, controlled by a bubble counter or a Brooks gas flowmeter, was applied. The oven was heated to 650 °C. After 12 h, the oven was cooled to 70 °C over 9 hours. The resulting activated catalyst was collected in a dry bottle and immediately transferred to a glove box.

### Disproportionation Reactions

A solution of 20.6 mg of triethylborane in hexane (14,2 wt%) in 247.7 mg of trans-decene-5 was made. This solution is hereinafter referred to as 'Solution A'

4 reaction mixtures were made by mixing Solution A with neat *trans*-decene-5 in the amounts indicated in Table 1. 40 mg of *n*-butylcyclohexane (a GC standard) was added to each reaction mixture.

112.5 mg of the activated catalyst was added to each reaction mixture and the mixture was then stirred at 120 °C for 1 hour before being allowed to cool to room temperature. The reaction mixture was removed from the catalyst and analysed by GC. The results are shown in Table 1.

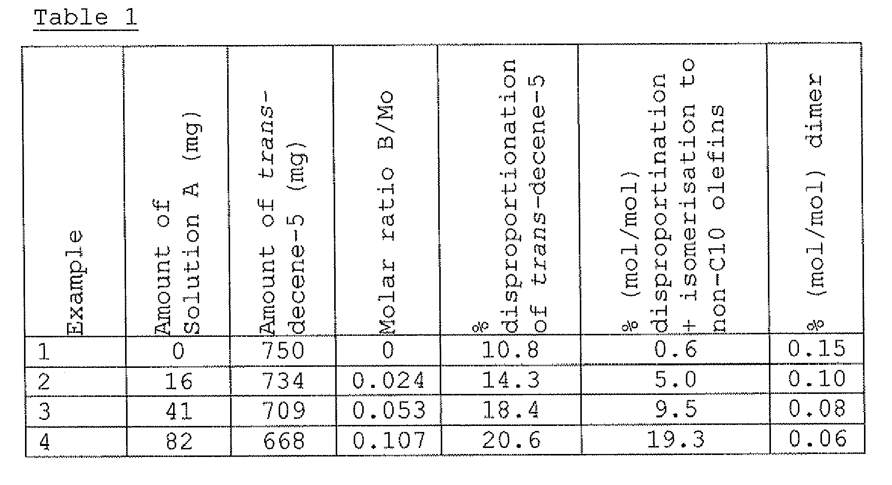

## Claims

1. A process for the disproportionation of olefinic hydrocarbons, said process comprising contacting at least one olefinic hydrocarbon with a catalyst comprising at least one of molybdenum and rhenium supported on an inorganic oxide support in the presence of a promoter selected from organoborane and organoaluminium compounds, wherein said process is carried out at a temperature in the range of from 100 to 200 °C.

2. A process as claimed in claim 1, wherein the promoter is an organoborane compound selected from the group consisting triethylborane, tri-sec-butylborane, tricyclohexylborane, 9-borabicyclo[3,3,1]nonane, diethylborane and mixtures thereof.

3. A process as claimed in claim 2, wherein the organoborane is selected from triethylborane and tri-sec-butylborane.

4. A process as claimed in any one of claims 1 to 3, wherein the temperature is in the range of from 110 to 150 °C.

5. A process as claimed in any one of claims 2 to 4, wherein the molar ratio of molybdenum and/or rhenium to organoborane is in the range of from 0.1 to 50.

6. A process as claimed in any one of claims 1 to 5, wherein the inorganic oxide support is selected from the group consisting of silica, alumina, silica-alumina, silica-magnesia, silica-titania, alumina-titania, alumina-magnesia, bora-alumina-silica, alumina-zirconia, thoria, silica-titania-zirconia and mixtures thereof.

7. A process as claimed in any one of claims 1 to 6, wherein said at least one olefinic hydrocarbon has a carbon number in the range of from C₂ to C₅₀.
